# EUROPEAN PATENT APPLICATION

(11) **EP 2 184 026 A1**
(43) Date of publication of application: **12.05.2010**
(21) Application number: 08764977.8
(22) Date of filing: 02.06.2008
(51) Int. Cl.: A61B 19/00

(54) **MEDICAL MANUAL SKILL DEVICE**

(30) Priority: 28.08.2007 JP 2007221686
(71) Applicant: Olympus Medical Systems Corp., Tokyo 151-0072 (JP)
(72) Inventor: UMEMOTO, Yoshitaka, Tokyo 151-0072 (JP); TAKAHASHI, Kazuhiko, Tokyo 151-0072 (JP)
(74) Representative: Beyer, Andreas
(86) International application number: PCT/JP2008/060164
(87) International publication number: WO 2009/028245

(57) **Abstract**

An active mechanism control apparatus includes a state detecting section which detects an output state of energy supplied to an energy treatment instrument by a status signal from an energy output apparatus. The state detecting section controls an energy output device, an active endoscope control section and an active treatment instrument control section based on the output state of the energy to the energy treatment instrument.

## Description

### Technical Field

The present invention relates to a minimally invasive medical manipulation apparatus, and particularly relates to a minimally invasive medical manipulation apparatus having an energy treatment instrument which is used under endoscopic observation.

### Background Art

Conventionally as minimally invasive medical manipulation, operations under endoscopic observation using endoscope images and operations under ultrasound observation using ultrasound images have been performed.

For example, Japanese Patent Application Laid-Open Publication No. 8-52153 discloses an electric scalpel device which guides a snare to a tip end of an endoscope through a forceps channel of the endoscope, passes a current to an affected part from the snare, and treats the affected part.

Further, Japanese Patent Application Laid-Open Publication No. 2000-312681 discloses an ultrasound operation system which drives a therapeutic ultrasound treatment instrument under ultrasound observation, and treats an affected part.

When energy treatment instruments such as the electric scalpel device and therapeutic ultrasound instrument as described above are used in the minimally invasive medical manipulation using the endoscope images and ultrasound images, a problem occurs in which the treatment energy at the time of drive of the energy treatment instruments becomes noise, and has an influence on the images of the endoscope images and ultrasound images.

Thus, for example, in the ultrasound operation system of the aforementioned Japanese Patent Application Laid-Open Publication No. 2000-312681, an art is disclosed in which when the ultrasound treatment transducer of a therapeutic ultrasound treatment instrument is driven, the output level of the ultrasound treatment transducer is reduced at the timing of drive of the ultrasound observation transducer, and thereby, the resistance to noise due to treatment energy is enhanced.

Meanwhile, in recent years, as shown in, for example, Japanese Patent Application Laid-Open Publication No. 6-114000, Japanese Patent Application Laid-Open Publication No. 2006-192201, or the like, an active endoscope and an active instrument tool having active functions capable of realizing maneuverability at desired degrees of freedom have been disclosed.

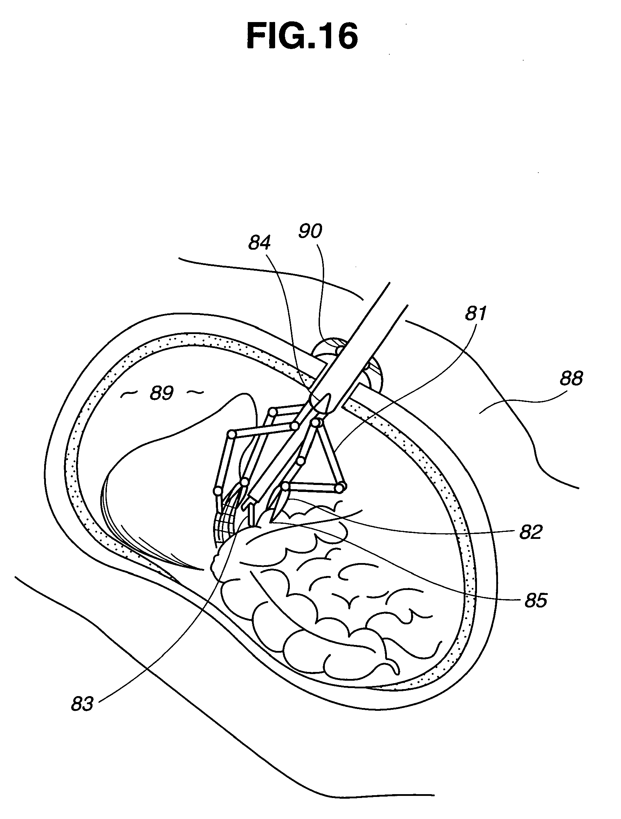

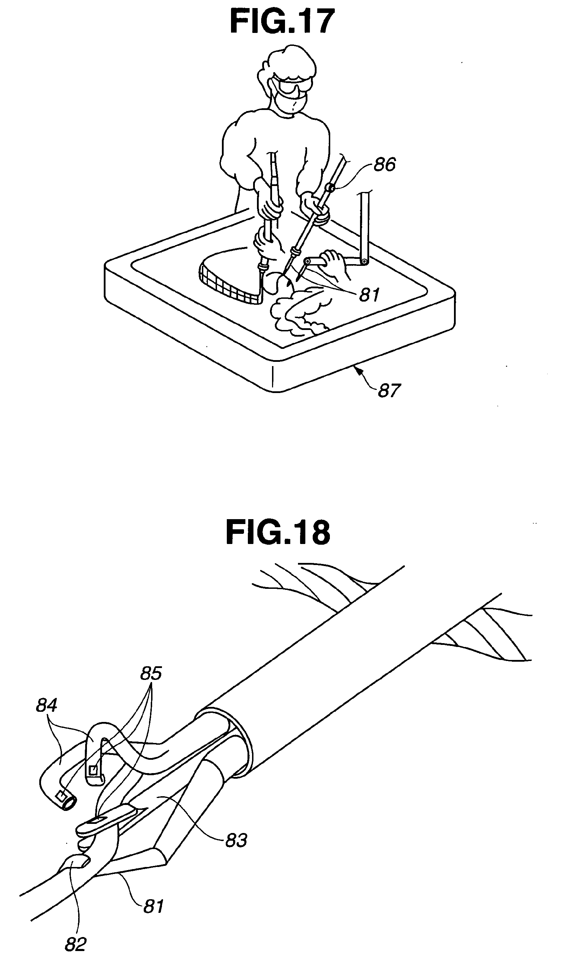

An example of an active endoscope and an active treatment instrument will be described with use of Figs. 16 to 18. Figs. 16 and 17 show each a schematic configuration of a minimally invasive intraperitoneal surgical operation apparatus which is used in an intraperitoneal operation under an endoscope. The intraperitoneal surgical operation apparatus is configured by an articulated micro manipulator 81, a micro gripper 82, and a ligation micro clip/micro suture instrument 83 as active treatment instruments, a micro stereoscopic endoscope 84 as an active endoscope, a micro tactile sensor 85, a remote control operation mechanism 86, a three-dimensional display device 87 and the like, as shown in Figs. 16 and 17, and an intraperitoneal operation is performed by the following method.

More specifically, an insufflating needle is inserted into an abdominal part 88 first, and the inside of an abdominal cavity 89 is insufflated. Next, the articulated micro manipulator 81 is inserted from the insufflated hole through a trocar 90, an organ is grasped with the micro gripper 82 with a tactile sensor, and the operative field is ensured.

Next, when a gall bladder is extracted, the gall bladder is lifted with the micro gripper 82. After a bile duct and a blood vessel are ligated with the micro clip 83 as shown in Fig. 18, they are cut with a high-frequency scalpel, and the gall bladder is extracted.

Observation of the inside of the abdominal cavity 89 is performed with the stereoscopic endoscope 84 mounted on the articulated micro manipulator 81.

At this time, an operator performs the operation with the remote control operation mechanism 86 while watching the stereoscopic image projected on the three-dimensional display device 87.

The minimally invasive intraperitoneal surgical operation apparatus of such a configuration only has to make one insertion hole in the abdominal part 88 of a patient, and therefore, has the advantage of having extremely low invasiveness to a patient. Further, an operator can perform an intraperitoneal surgical operation while watching the stereoscopic image of the inside of the abdominal cavity 89, and therefore, the operator can perform an operation with the sense similar to a surgical operation by abdominal section.

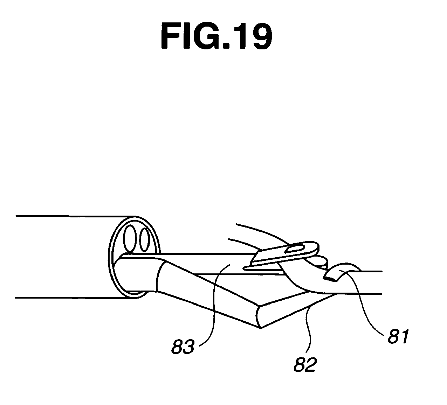

As shown in Fig. 19, the aforementioned active treatment instruments (the articulated micro manipulator 81, the micro gripper 82, and the ligation micro clip/micro suture instrument 83) can be inserted through the treatment instrument channels of the conventional (existing) endoscopes, and the active treatment instruments can be protruded from the distal end to perform treatment of an affected part.

However, in the minimally invasive medical manipulation using the aforementioned endoscope images and ultrasound images, a still image of an affected part needs to be stored during manipulation, but if storage of a still image is carried out during use of the energy treatment instrument, the conventional stored image is likely to be an image influenced by noise, and there is a problem that storage of optimal and proper images is difficult.

Further, in order to use the energy treatment instrument during manipulation using the aforementioned active endoscope or the active treatment instruments, the noise resistance of the active endoscope or the active treatment instruments needs to be enhanced. However, the active endoscope or the active treatment instruments is or are required to be reduced in the size of the active function part requiring a complicated mechanism and control, and therefore, there are many problems in realization of enhancement of noise resistance.

The present invention is made in view of the above described circumstances, and has an object to provide a medical manipulation apparatus which can enhance noise resistance in energy treatment easily at low cost.

### Disclosure of Invention

### Means for Solving the Problem

A medical manipulation apparatus of the present invention is configured by including
remote active treatment means including a remote treatment instrument remotely treating a subject,
active control means controlling the remote active treatment means,
energy treatment means performing energy treatment for the subject, and
control means detecting an energy output state of the energy treatment means, and changing active control of at least the remote active treatment means based on the energy output state.

### Brief Description of the Drawings

Fig. 1 is a configuration diagram showing a configuration of an endoscope system apparatus which is a medical manipulation apparatus according to embodiment 1 of the present invention;
Fig. 2 is a first waveform chart showing energy output outputted by an energy output device of Fig. 1;
Fig. 3 is a second waveform chart showing the energy output outputted by the energy output device of Fig. 1;
Fig. 4 is a third waveform chart showing the energy output outputted by the energy output device of Fig. 1;
Fig. 5 is a flowchart explaining an operation of the endoscope system apparatus of Fig. 1;
Fig. 6 is a configuration diagram showing a configuration of an endoscope system apparatus which is a medical manipulation apparatus according to embodiment 2 of the present invention;
Fig. 7 is a flowchart explaining an operation of the endoscope system apparatus of Fig. 6;
Fig. 8 is a configuration diagram showing a configuration of an endoscope system apparatus which is a medical manipulation apparatus according to embodiment 3 of the present invention;
Fig. 9 is a first flowchart explaining an operation of the endoscope system apparatus of Fig. 8;
Fig. 10 is a second flowchart explaining the operation of the endoscope system apparatus of Fig. 8;
Fig. 11 is a configuration diagram showing a configuration of an endoscope system apparatus which is a medical manipulation apparatus according to embodiment 4 of the present invention;
Fig. 12 is a first flowchart explaining an operation of the endoscope system apparatus of Fig. 11;
Fig. 13 is a first flowchart explaining the operation of the endoscope system apparatus of Fig. 11;
Fig. 14 is a configuration diagram showing a configuration of an endoscope system apparatus which is a medical manipulation apparatus according to embodiment 5 of the present invention;
Fig. 15 is a flowchart explaining an operation of the endoscope system apparatus of Fig. 14;
Fig. 16 is a first view explaining a conventional intraperitoneal surgical operation apparatus;
Fig. 17 is a second view explaining the conventional intraperitoneal surgical operation apparatus;
Fig. 18 is a third view explaining the conventional intraperitoneal surgical operation apparatus; and
Fig. 19 is a fourth view explaining the conventional intraperitoneal surgical operation apparatus.

### Best Mode for Carrying Out the Invention

Hereinafter, embodiments of the present invention will be described with reference to the drawings.

### (Embodiment 1)

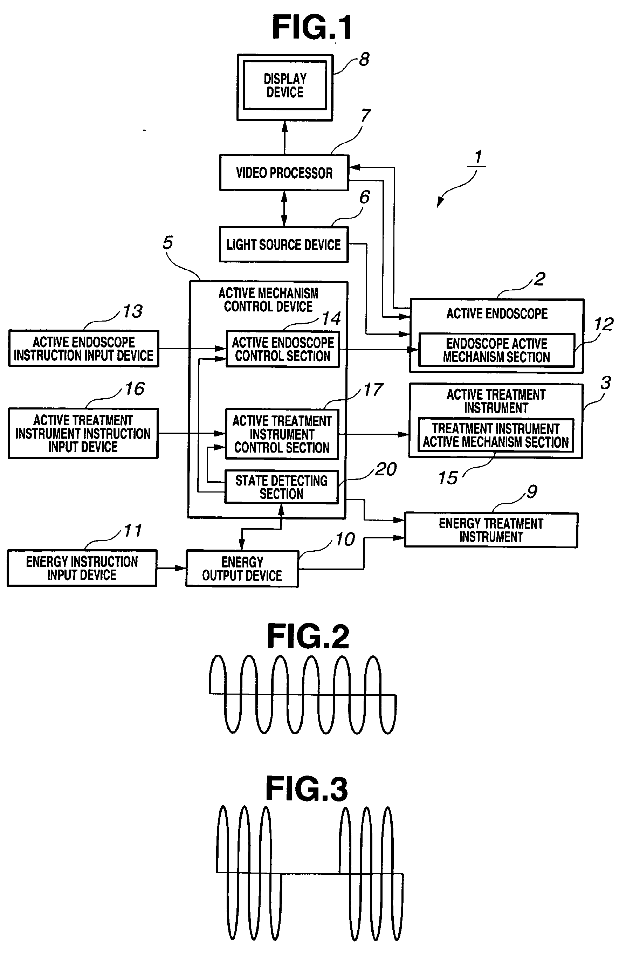

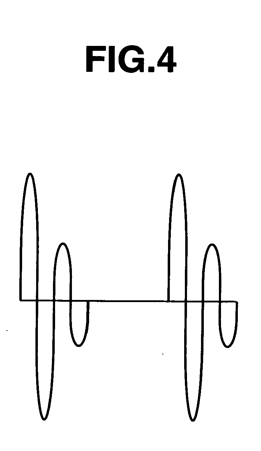

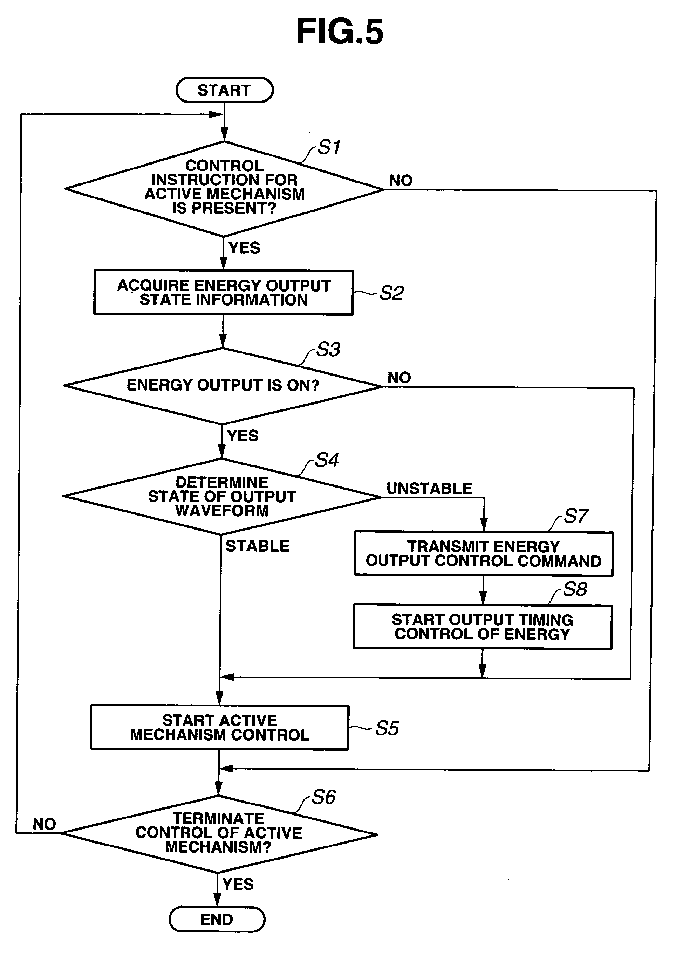

Figs. 1 to 5 relate to embodiment 1 of the present invention. Fig. 1 is a configuration diagram showing a configuration of an endoscope system apparatus which is a medical manipulation apparatus. Fig. 2 is a first waveform chart showing energy output outputted by an energy output device of Fig. 1. Fig. 3 is a second waveform chart showing the energy output outputted by the energy output device of Fig. 1. Fig. 4 is a third waveform chart showing the energy output outputted by the energy output device of Fig. 1. Fig. 5 is a flowchart explaining an operation of the endoscope system apparatus of Fig. 1.

### (Configuration)

As shown in Fig. 1, an endoscope system apparatus 1 which is a medical manipulation apparatus of the present embodiment is configured by including an active endoscope 2, an active treatment instrument 3, an active mechanism control device 5 as control means, a light source device 6, a video processor 7 as endoscope signal processing means, a display device 8, an energy treatment instrument 9, and an energy output device 10.

The energy output device 10 includes an energy instruction input device 11, and the energy instruction input device 11, the energy treatment instrument 9 and the energy output device 10 configure energy treatment means such as an electric scalpel device and an ultrasound treatment device, which performs treatment modes (hemostasis, dissection or coagulation mode or the like) for an affected part.

Further, the active endoscope 2 is configured by including an image pickup device (not illustrated) such as a CCD or C-MOS sensor or the like at a distal end, for example, and having an endoscope active mechanism section 12 as remote active bending means having an active function having a desired degree of freedom. The endoscope active mechanism section 12 is controlled so as to perform a desired operation by an active endoscope control section 14 of the active mechanism control device 5 based on an instruction signal from an active endoscope instruction input device 13 such as a joy stick, for example, which is connected to the active mechanism control device 5.

Similarly, the active treatment instrument 3 is configured by including a small-diameter insertion section (not illustrated) which can be inserted through the treatment instrument channel (not illustrated) of the active endoscope 2, for example, and can be protruded from a distal end, and having a treatment instrument active mechanism section 15 as remote active treatment means having an active function having a desired degree of freedom. The treatment instrument active mechanism section 15 is controlled to perform a desired operation by an active treatment instrument control section 17 of the active mechanism control device 5 based on an instruction signal from an active treatment instrument instruction input device 16 such as a joy stick, for example, which is connected to the active mechanism control device 5.

The light source device 6 is a light source section which supplies illumination light to the active endoscope 2. Further, the video processor 7 is an image processing section which drives the image pickup device (not illustrated) of the active endoscope 2, performs signal processing for an image pickup signal and displays an endoscope image on the display device 8.

Further, the active mechanism control device 5 includes a state detecting section 20 which detects an output state of energy supplied to the energy treatment instrument 9 by a status signal from the energy output device 10.

The state detecting section 20 controls the energy output device 10, the active endoscope control section 14 and the active treatment instrument control section 17 based on the output stage of the energy to the energy treatment instrument 9. The details of control of the state detecting section 20 will be described later.

### (Operation)

The operation of the endoscope system apparatus 1 of the present embodiment thus configured will be described.

The energy output device 10 supplies energy output as shown in Figs. 2 to 4 to the energy treatment instrument 9 in accordance with the treatment mode to an affected part.

The output waveform of Fig. 2 is a high-frequency waveform which continuously changes, and is a waveform (hereinafter, called an unstable state waveform) in which the changing speed of the waveform (differential value of the waveform) continuously changes quickly, and the waveform period of the state where the differential value of the waveform is a predetermined value or less (=the state in which the changing speed of the waveform is the predetermined speed or lower) is not present for a predetermined period.

Meanwhile, the waveforms of Figs. 3 and 4 are the waveforms in which energy is intermittently outputted, and are waveforms (hereinafter, called a stable state waveform) in which the period in which energy is not outputted in the waveforms for a predetermined period (the differential value of the waveform=0) is present.

The endoscope system apparatus 1 inserts the active endoscope 2, the active treatment instrument 3 and the energy treatment instrument 9 into a body cavity and carries out manipulation under endoscopic observation.

Subsequently, when treatment to an affected part is started by the energy instruction input device 11, the active mechanism control device 5 determines whether or not a control instruction for the active mechanism is given by an operator by detecting the instruction signal from the active endoscope instruction input device 13 or the active treatment instrument instruction input device 16 in the active endoscope control section 14 or the active treatment instrument control section 17 in step S1 as shown in Fig. 5.

When the control instruction for the active mechanism is present, the active mechanism control device 5 acquires the energy output state information from the state detecting section 20 by the status signal from the energy output device 10 in step S2.

More specifically, in step S2, the state detecting section 20 detects first information of whether energy output is executed from the energy output device 10 or not, and second information of whether the state waveform of the output waveform when the energy is outputted is a stable state waveform or an unstable state waveform as the energy output state information, by the status signal.

The state detecting section 20 detects the second information of the energy output state information as the information based on the changing speed of the waveform, but the second information is not limited to this. The second information may be the information indicating whether or not the output is of a predetermined amplitude or more (for example, the waveform of a predetermined amplitude or more = unstable state waveform, the waveform of an amplitude less than the predetermined amplitude = stable state waveform), and may be the information combining the changing speed and amplitude of the waveform.

Subsequently, in step S3, the active mechanism control device 5 determines whether or not the energy output device 10 is under energy output (energy output is ON) based on "the information whether or not energy output is executed from the energy output device 10" which is the first information of the energy output state information detected by the state detecting section 20.

Upon determining that it is under energy output (energy output is ON), the active mechanism control device 5 determines whether the state waveform of the output waveform is a stable state waveform or an unstable state waveform by the second information of the energy output state information detected by the state detecting section 20 in step S4.

Upon determining that the state waveform of the output waveform is a stable state waveform, the active mechanism control device 5 controls the active endoscope control section 14 or the active treatment instrument control section 17, and starts control of the active mechanism of the active endoscope 2 or the active treatment instrument 3 in step 5. If it is determined that it is not under energy output (energy output is ON) in the above described step S3, the active mechanism control device 5 still proceeds to step S5.

Subsequently, in step S6, the active mechanism control device 5 repeats the processing of the above described steps S1 to S5 until the active mechanism control device 5 detects termination of control of the active mechanism (termination of treatment by the endoscope system apparatus 1).

More specifically, when it is not under energy output (energy output is ON), or when the state waveform of the output waveform of the energy output is a stable state waveform, noise which is likely to have an influence on control of the active mechanism of the active endoscope 2 or the active treatment instrument 3 does not occur from the energy output device 10, and therefore, the active mechanism control device 5 executes control of the active mechanism of the active endoscope 2 or the active treatment instrument 3 in step S5.

On the other hand, upon determining that the state waveform of the output waveform is an unstable state waveform in step S4, the active mechanism control device 5 transmits an energy output control command to the energy output device 10 in step S7.

The energy output control command is a command for ordering stoppage of energy output or restriction of the output level for a certain predetermined period to the energy output device 10. When the energy output device 10 receives the energy output control command from the active mechanism control device 5, the energy output device 10 executes stoppage of energy output or restriction of the output level for a certain predetermined period.

When the active mechanism control device 5 transmits the energy output control command to the energy output device 10, the active mechanism control device 5 starts timing control of the period of stoppage of energy output or restriction of the output level for a certain predetermined period in the energy output device 10 in step S8, and proceeds to step S5.

More specifically, upon determining that the state waveform of the output waveform is an unstable state waveform, the active mechanism control device 5 performs stoppage of the energy output of the energy output device 10 or restriction of the output level for a certain predetermined period, and executes control of the active mechanism of the active endoscope 2 or the active treatment instrument 3 based on the timing of the certain predetermined period in step S5.

### (Effect)

Thus, in the present embodiment, the state detecting section 20 detects the energy output state information, whereby when it is not under energy output (energy output is ON), or when the state waveform of the output waveform of the energy output is a stable state waveform, noise which is likely to exert an influence on control of the active mechanism of the active endoscope 2 or the active treatment instrument 3 does not occur from the energy output device 10, and therefore, reliable and stable control of the active mechanism of the active endoscope 2 or the active treatment instrument 3 can be performed.

Further, the state detecting section 20 detects the energy output state information, whereby even when the state waveform of the output waveform is an unstable state waveform, noise which is likely to exert an influence on the control of the active mechanism of the active endoscope 2 or the active treatment instrument 3 does not occur from the energy output device 10 since stoppage of energy output of the energy output device 10 or restriction of the output level is performed for a certain predetermined period, and reliable and stable control of the active mechanism of the active endoscope 2 or the active treatment instrument 3 can be performed based on the timing of the certain predetermined period.

The endoscope system apparatus 1 of the present embodiment can perform control of the active mechanism of the active endoscope 2 or the active treatment instrument 3 reliably and stably without receiving the influence of the energy output device 10 by the processing of the aforementioned active mechanism control device 5, and therefore, has the effect of being capable of enhancing noise resistance in the energy treatment easily at low cost.

In the present embodiment, the state detecting section 20 detects the state specified based on the differential value of the output waveform as an example of the energy output state, but the state is not limited to this, and for example, the state detecting section 20 may detect the state specified based on at least any one of the above described differential value, the integrated value, the amplitude value and the frequency of the output waveform as the energy output state.

### (Embodiment 2)

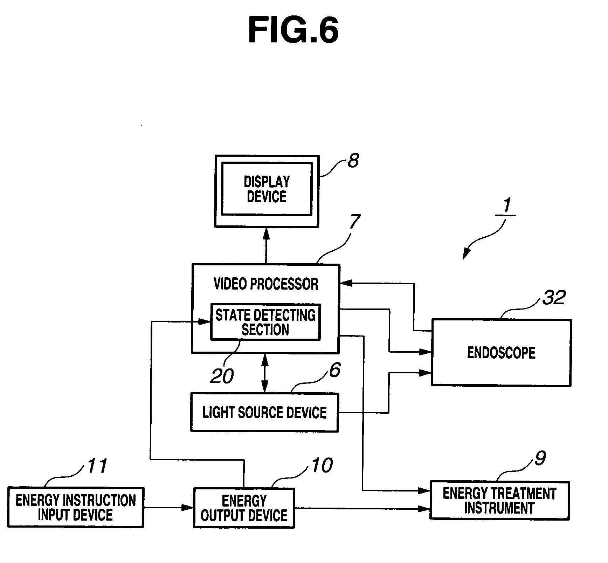

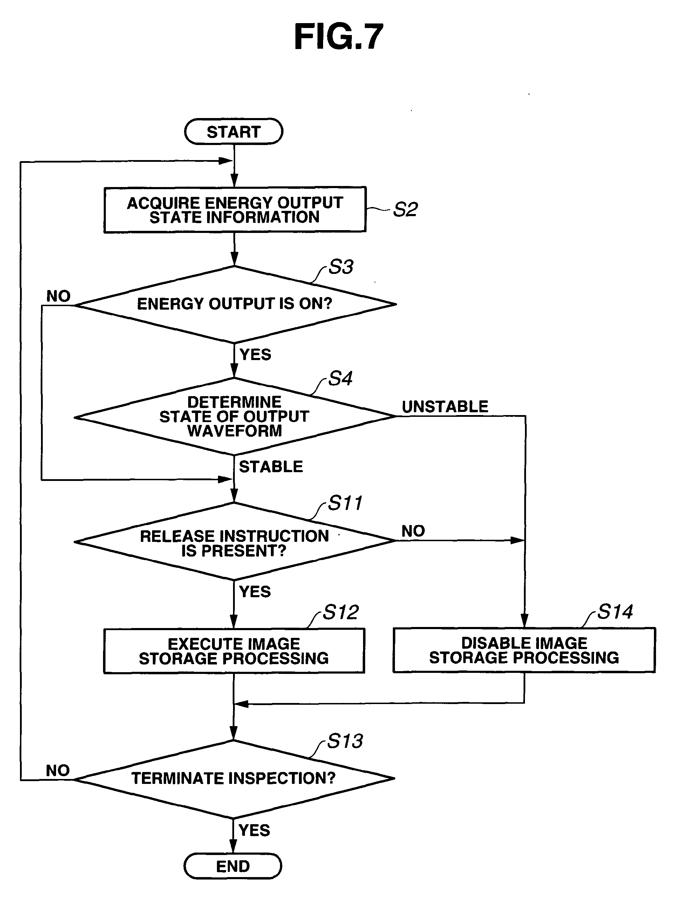

Figs. 6 and 7 relate to embodiment 2. Fig. 6 is a configuration diagram showing a configuration of an endoscope system apparatus which is a medical manipulation apparatus. Fig. 7 is a flowchart explaining an operation of the endoscope system apparatus of Fig. 6.

Embodiment 2 is substantially the same as embodiment 1, and therefore, only the different point will be described. The same components are assigned with the same reference numerals, and description thereof will be omitted.

### (Configuration)

As shown in Fig. 6, the present embodiment does not have the active mechanism control device 5, and is the embodiment of an endoscope system apparatus 31 which carries out the treatment by the conventional ordinary endoscope (electronic endoscope) 32 instead of the active endoscope 2 and the active treatment instrument 3, and the state detecting section 20 is provided in the video processor 7. The other components are the same as those of embodiment 1.

### (Operation)

An operation of the endoscope system apparatus 31 of the present embodiment thus configured will be described.

Ordinarily, the endoscope 32 has a release function for storing a still image of the endoscope image which the endoscope 32 picks up. However, when a still image is stored by executing the release function during output of the energy of the energy output device 10, the influence of the noise due to energy output of the energy output device 10 is reflected on the stored still image, and a desired still image is not likely to be stored with desired image quality, but in the present embodiment, storage of a still image is executed by executing the processing as shown in Fig. 7.

More specifically, as shown in Fig. 7, after the video processor 7 executes steps S2 to S4 described in embodiment 1 in place of the active mechanism control device 5, if the video processor 7 determines that the state waveform of the output waveform is a stable state waveform in step S4, the video processor 7 proceeds to step S11.

In step S11, the video processor 7 determines whether or not the release switch not illustrated of the endoscope 32 is operated and a release instruction is given. Upon determining that the release instruction is given, the video processor 7 executes processing of storing the still image of the endoscope image by known release processing in step S12. Subsequently, the video processor 7 repeats the processing of steps S2 to S4, S11 and S12 until the video processor 7 detects termination of inspection in step S13.

On the other hand, upon determining that the state waveform of the output waveform is an unstable state waveform in step S4, the video processor 7 proceeds to step S14.

In step S14, the video processor 7 disables (unable to perform storage processing) storage processing of the still image of the endoscope image, and proceeds to step S13.

### (Effect)

Thus, in the present embodiment, the energy output state of the energy output device 10 is detected at the time of image storage, and executable/unexecutable of the image storage is controlled in accordance with the output stage. Therefore, the present embodiment has the effect of being capable of reliably and stably executing release processing without receiving the influence of the energy output device 10, and being capable of enhancing noise resistance in the energy treatment easily at low cost.

### (Embodiment 3)

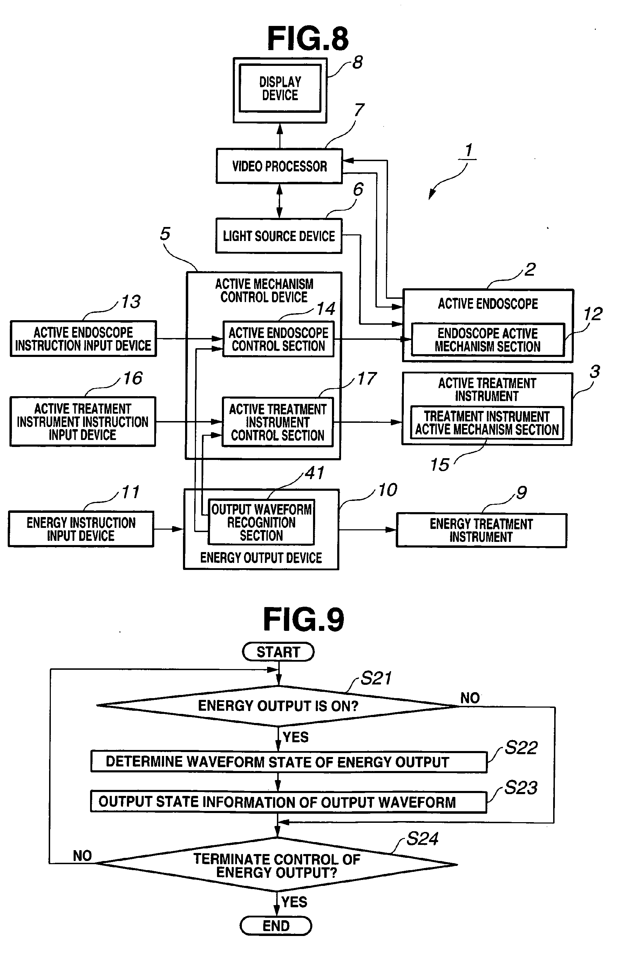

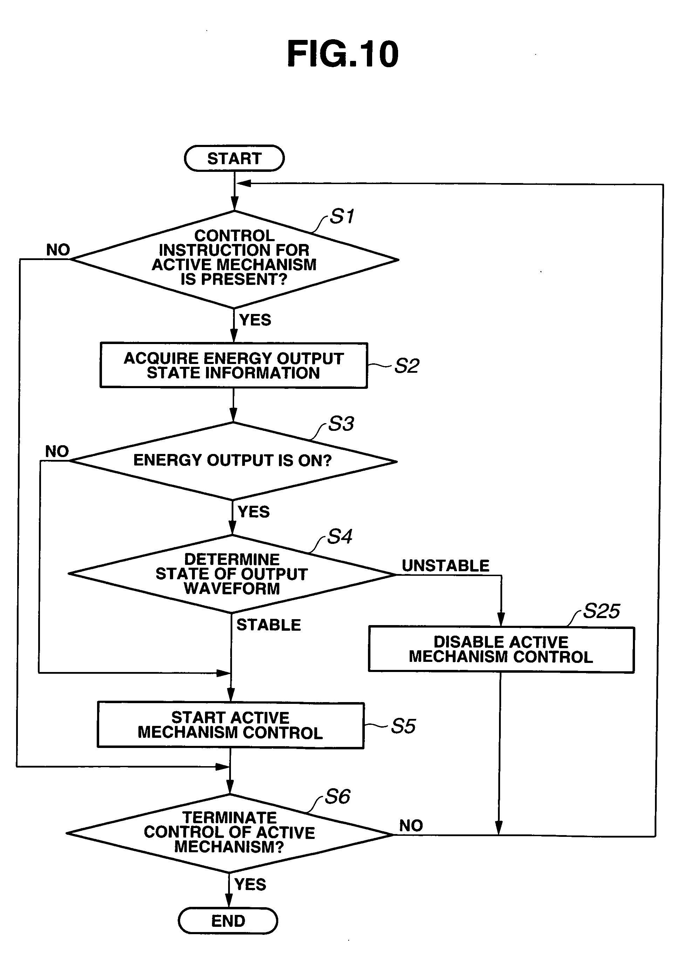

Figs. 8 to 10 relate to embodiment 3 of the present invention. Fig. 8 is a configuration diagram showing a configuration of an endoscope system apparatus which is a medical manipulation apparatus. Fig. 9 is a first flowchart explaining an operation of the endoscope system apparatus of Fig. 8. Fig. 10 is a second flowchart explaining the operation of the endoscope system apparatus of Fig. 8.

Embodiment 3 is substantially the same as embodiment 1, and therefore, only the different point will be described. The same components are assigned with the same reference numerals, and description thereof will be omitted.

### (Configuration)

In the present embodiment, as shown in Fig. 8, an output waveform recognition section 41 is provided in the energy output device 10 in place of the state detecting section 20 of the active mechanism control device 5. The output waveform recognition section 41 analyzes the output waveform of the energy to the energy treatment instrument 9, and recognizes the energy output state information described in embodiment 1. The output waveform recognition section 41 outputs the recognized energy output state information to the active mechanism control device 5. The active mechanism control device 5 controls the active endoscope control section 14 or the active treatment instrument control section 17 based on the received energy output state information. The other components are the same as those of embodiment 1.

### (Operation)

In the present embodiment, as shown in Fig. 9, the output waveform recognition section 41 determines whether or not the output of the energy to the energy treatment instrument 9 is ON in step S21.

Next, when the output of the energy is ON, the output waveform recognition section 41 analyzes the output waveform of the energy to the energy treatment instrument 9 in step S22, and determines the waveform state (a stable state waveform or an unstable state waveform). When the output of the energy is not ON, the present processing proceeds to step S24.

Subsequently, the output waveform recognition section 41 outputs the determined waveform state (a stable state waveform or an unstable state waveform) to the active mechanism control device 5 as the energy output state information in step S23. The output waveform recognition section 41 repeats the processing until the output waveform recognition section 41 detects termination of the energy output control in step S24.

Meanwhile, the active mechanism control device 5 executes the processing of steps S1 to S6 described in embodiment 1 based on the energy output state information from the output waveform recognition section 41 as shown in Fig. 10.

In the present embodiment, upon determining that the state waveform of the output waveform is an unstable state waveform in step S4, the active mechanism control device 5 disables the active mechanism control in step S25 to return to step S1.

### (Effect)

Thus, in the embodiment, the output waveform recognition section 41 analyzes/recognizes energy output, whereby control of the active mechanism of the active endoscope 2 or the active treatment instrument 3 can be performed reliably and stably without receiving the influence of the energy output device 10 as in embodiment 1, and therefore, the embodiment has the effect of being capable of enhancing noise resistance in the energy treatment easily at low cost.

### (Embodiment 4)

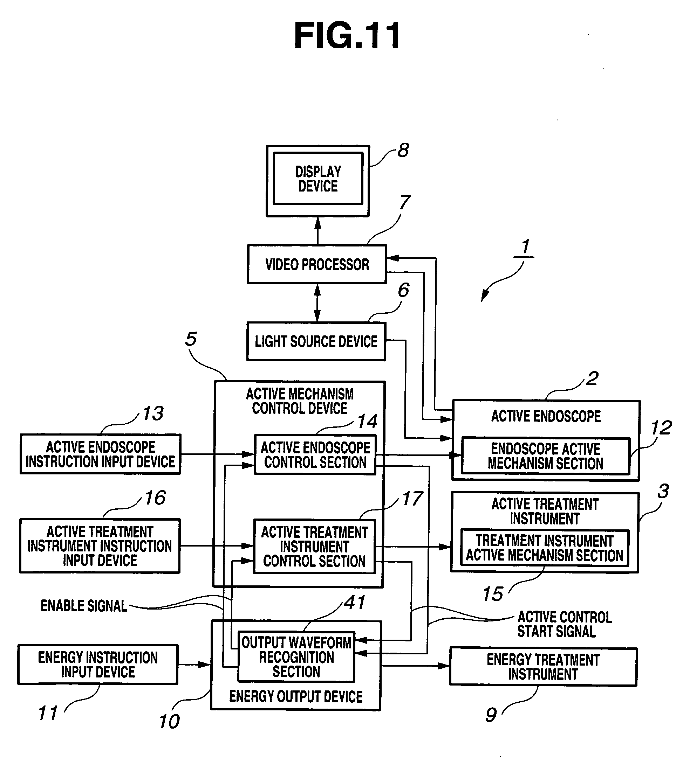

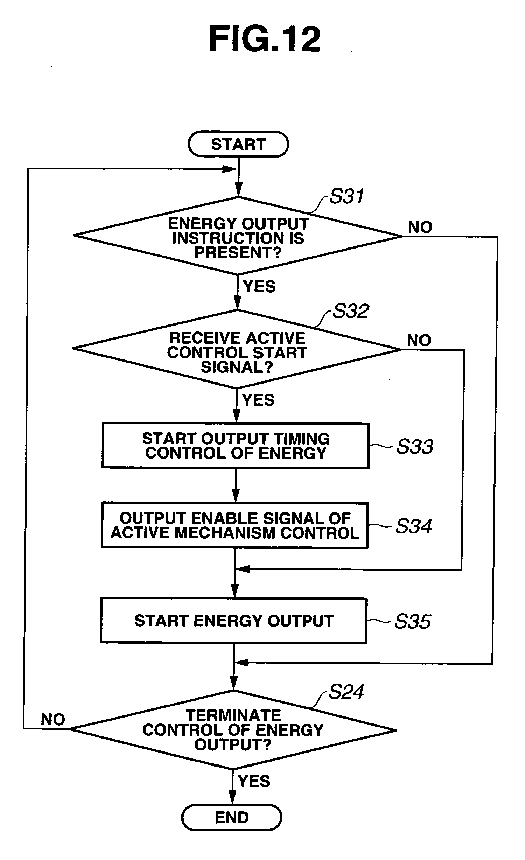

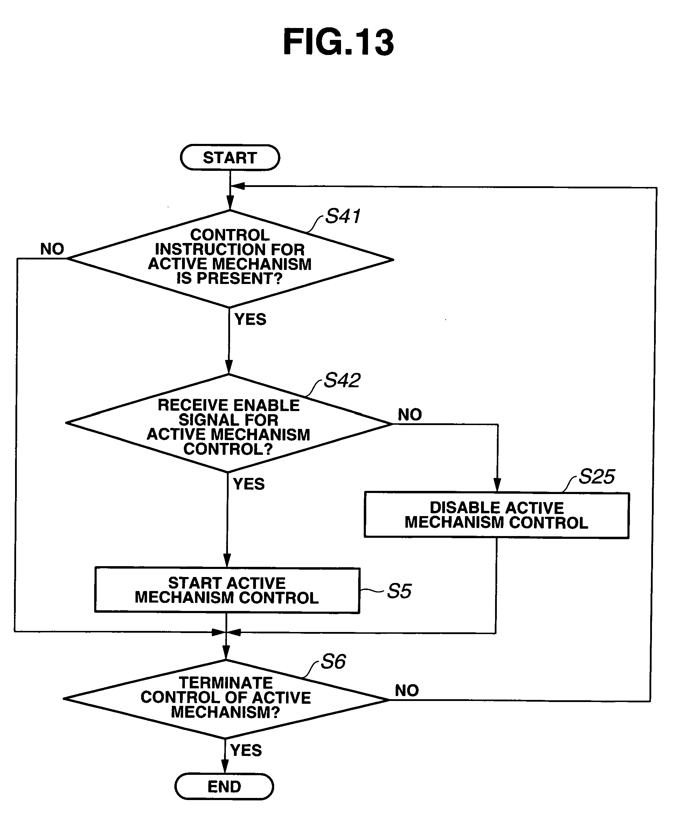

Figs. 11 to 13 relate to embodiment 4 of the present invention. Fig. 11 is a configuration diagram showing a configuration of an endoscope system apparatus which is a medical manipulation apparatus. Fig.12 is a first flowchart explaining an operation of the endoscope system apparatus of Fig. 11. Fig. 13 is a second flowchart explaining the operation of the endoscope system apparatus of Fig. 11.

Embodiment 4 is substantially the same as embodiment 3, and therefore, only the different point will be described. The same components are assigned with the same reference numerals and description thereof will be omitted.

### (Configuration)

In the present embodiment, as shown in Fig. 11, the output waveform recognition section 41 in the energy output device 10 is configured so as to receive an active control start signal indicating start of active control by the active endoscope control section 14 or the active treatment instrument control section 17 from the active endoscope control section 14 or the active treatment instrument control section 17. The output waveform recognition section 41 executes timing control of energy output by the active control start signal. The other components are the same as those in embodiment 3.

### (Operation)

In the present embodiment, as shown in Fig. 12, the output waveform recognition section 41 of the energy output device 10 determines whether or not the instruction of energy output is given in the energy instruction input device 11 in step S31, and when the instruction of energy output is given, the process proceeds to step S32. When the instruction of energy output is not given, the process proceeds to step S24.

In step S32, the output waveform recognition section 41 of the energy output device 10 determines whether or not the output waveform recognition section 41 receives an active control start signal from the active endoscope control section 14 or the active treatment instrument control section 17, and upon determining that it received the active control start signal, the output waveform recognition section 41 proceeds to step S33. When the output waveform recognition section 41 does not receive the active control start signal, the output waveform recognition section 41 proceeds to step S35.

In step S33, the output waveform recognition section 41 of the energy output device 10 starts timing control of energy output at the time of output of an unstable state waveform, and in step S34, the output waveform recognition section 41 outputs an enabling signal for the active mechanism control to the active endoscope control section 14 or the active treatment instrument control section 17. More specifically, the output waveform recognition section 41 stops energy output for only a predetermined period in the mode in which the unstable state waveform is outputted based on the reception of the active control start signal (step S33), and outputs an enable signal for active mechanism control to the active endoscope control section 14 or the active treatment instrument control section 17 for the predetermined period of stoppage of the output (step S34).

After the lapse of the predetermined period of the output stoppage, the output waveform recognition section 41 starts energy output in step S35, and proceeds to step S24 described in embodiment 3.

Meanwhile, the active endoscope control section 14 or the active treatment instrument control section 17 determines whether or not input of the control instruction of the active mechanism is given from the active endoscope instruction input device 13 or the active treatment instrument instruction input device 16 in step S41 as shown in Fig. 13. When the control instruction of the active mechanism is given, the active endoscope control section 14 or the active treatment instrument control section 17 outputs the active control start signal to the output waveform recognition section 41 of the energy output device 10, and proceeds to step S42. When an instruction for energy output is not given, the process proceeds to step S6.

In step S42, the active endoscope control section 14 or the active treatment instrument control section 17 determines whether or not it receives the enable signal of the active mechanism control from the output waveform recognition section 41 of the energy output device 10. When receiving the enable signal for active mechanism control, the active endoscope control section 14 or the active treatment instrument control section 17 executes the processing of step S5 described in embodiment 1 during the reception period, and when receiving no enable signal for the active mechanism control, the active endoscope control section 14 or the active treatment instrument control section 17 executes the processing of step S25 described in embodiment 3, and proceeds to step S6 described in embodiment 1.

More specifically, in the present embodiment, when the active endoscope control section 14 or the active treatment instrument control section 17 controls the active mechanism, the active endoscope control section 14 or the active treatment instrument control section 17 outputs an active control start signal to the energy output device 10.

Subsequently, the energy output device 10 stops output of the energy of the unstable state waveform for a predetermined period (for example, time required by control of the active endoscope control section 14 or the active treatment instrument control section 17 is required) during output of the energy of an unstable state waveform based on the active control start signal.

The time required for one command control of the active endoscope control section 14 or the active treatment instrument control section 17 is in the order of ns or µs, and therefore, even if the energy output is stopped for this time period, energy treatment is not influenced.

### (Effect)

As described above, in the present embodiment, as in embodiment 3, the output waveform recognition section 41 analyzes/recognizes the energy output, and controls the timing of the energy output based on the active control start signal, whereby, control of the active mechanism of the active endoscope 2 or the active treatment instrument 3 can be performed reliably and stably without receiving the influence of the energy output device 10. Therefore, the present embodiment has the effect of being capable of enhancing noise resistance in the energy treatment easily at low cost.

### (Embodiment 5)

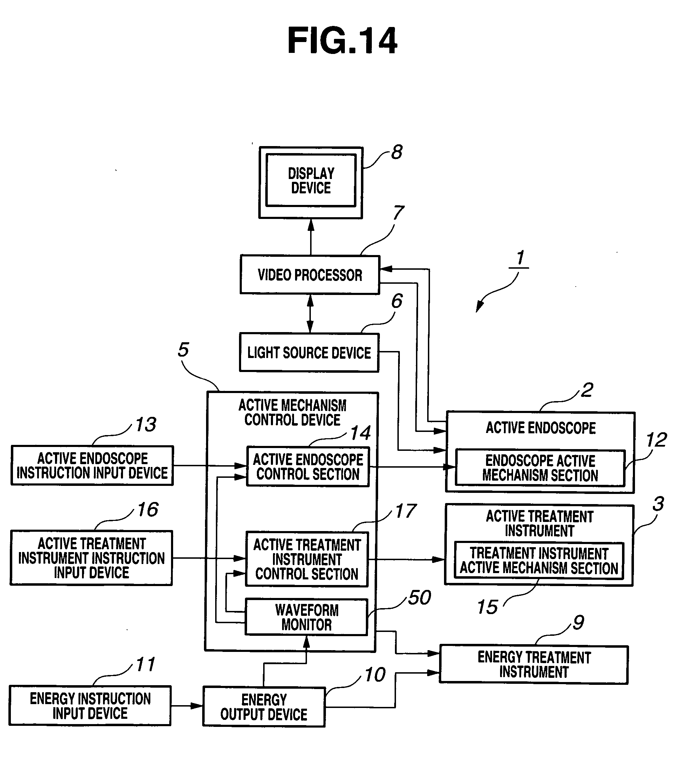

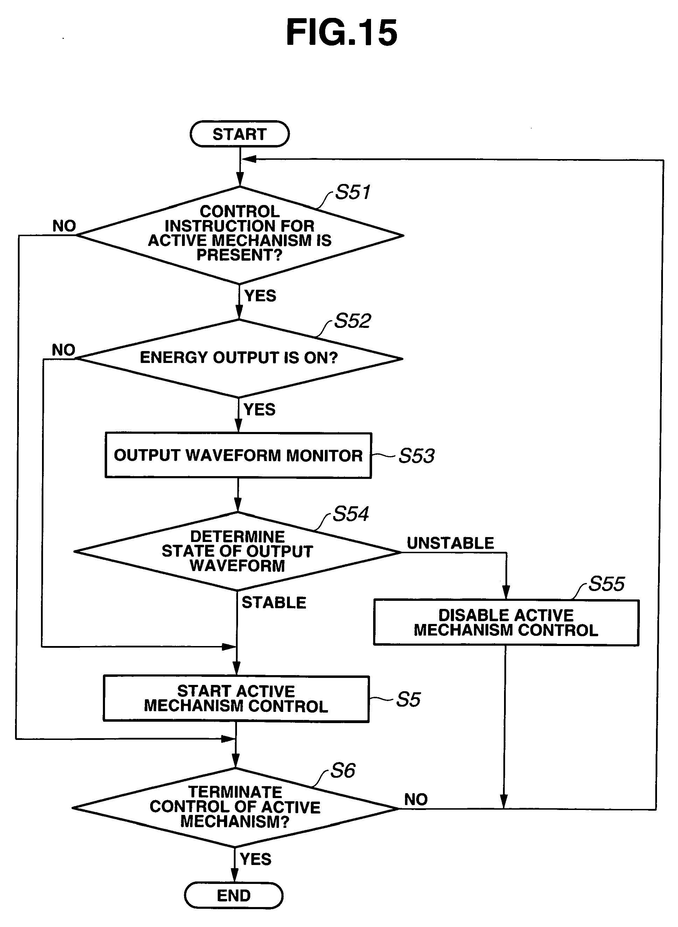

Figs. 14 and 15 relate to embodiment 5 of the present invention. Fig. 14 is a configuration diagram showing a configuration of an endoscope system apparatus which is a medical manipulation apparatus. Fig. 15 is a flowchart explaining an operation of the endoscope system apparatus of Fig. 14.

Embodiment 5 is substantially the same as embodiment 1, and therefore, only the different point will be described. The same components are assigned with the same reference numerals and description thereof will be omitted.

### (Configuration)

As shown in Fig. 14, the active mechanism control device 5 is configured by including a waveform monitor section 50 which monitors the output waveform of the energy output device 10 in place of the state detecting section 20. The waveform monitor section 50 monitors the output waveform of the energy output device 10, performs waveform analysis processing, determines the waveform state (a stable state waveform or an unstable state waveform) of the output waveform, and controls the active endoscope control section 14 or the active treatment instrument control section 17 in accordance with the waveform state as described in embodiment 1. The other components are the same as those in embodiment 1.

### (Operation)

In the present embodiment, the active mechanism control device 5 detects the instruction signal from the active endoscope instruction input device 13 or the active treatment instrument instruction input device 16 in the active endoscope control section 14 or the active treatment instrument control section 17 in step S51 as shown in Fig. 15, and thereby, determines whether or not the control instruction for the active mechanism is given by an operator.

When the control instruction for the active mechanism is given, the waveform monitor section 50 of the active mechanism control device 5 determines whether or not the energy output is executed from the energy output device 10 in step S52. When the energy output is executed, the waveform monitor section 50 monitors the energy output waveform in step S53, and analyzes the monitored energy output waveform to determine the waveform state (a stable state waveform or an unstable state waveform) of the output waveform in step S54.

Subsequently, based on the energy output state in the waveform monitor section 50, the active mechanism control device 5 executes the processing of steps S5 to S6 described in embodiment 1.

In the present embodiment, upon determining that the state waveform of the output waveform is an unstable state waveform in step S54, the active mechanism control device 5 disables the active mechanism control in step S55 and returns to step S51.

### (Effect)

As above, in the present embodiment, as in embodiment 1, control of the active mechanism of the active endoscope 2 or the active treatment instrument 3 can be performed reliably and stably without receiving the influence of the energy output device 10, and therefore, the present embodiment has the effect of being capable of enhancing noise resistance in energy treatment easily at low cost.

The present invention is not limited to the aforementioned embodiments, and various changes, modifications and the like can be made in the range without changing the gist of the present invention.

The present application is based upon and claims the benefit of priority from the prior Japanese Patent Application No. 2007-221686, filed on August 28, 2007; the entire contents of which are incorporated in the description and claims of the present application by reference.

## Claims

1. A medical manipulation apparatus, comprising:
remote active treatment means including a remote treatment instrument remotely treating a subject;
active control means controlling the remote active treatment means;
energy treatment means performing energy treatment for the subject; and
control means detecting an energy output state of the energy treatment means, and changing active control of at least the remote active treatment means based on the energy output state.

2. A medical manipulation apparatus, comprising:
an endoscope having image pickup means picking up an image of a subject;
endoscope signal processing means driving the image pickup means, and performing signal processing of an image pickup signal from the image pickup means;
energy treatment means performing energy treatment for the subject; and
control means detecting an energy output state of the energy treatment means, and changing image pickup control of at least the image pickup means based on the energy output state.

3. A medical manipulation apparatus, comprising:
an endoscope having image pickup means picking up an image of a subject;
endoscope signal processing means driving the image pickup means, and performing signal processing of an image signal from the image pickup means;
remote active treatment means including a remote treatment instrument remotely treating the subject;
active control means controlling the remote active treatment means;
energy treatment means performing energy treatment for the subject; and
control means detecting an energy output state of the energy treatment means, and changing at least one of image pickup control of the image pickup means and active control of the remote active treatment means based on energy output state.

4. The medical manipulation apparatus according to claim 1, further comprising an endoscope having image pickup means picking up an image of the subject.

5. The medical manipulation apparatus according to any one of claims 2 to 4, wherein
the endoscope has remote active bending means remotely bending an insertion section; and
the control means detects the energy output state of the energy treatment means, and changes active control of the remote active bending means based on the energy output state.

6. The medical manipulation apparatus according to any one of claims 1 to 5, wherein
the control means changes the energy output state of the energy treatment means based on the energy output state.

7. The medical manipulation apparatus according to any one of claims 1 to 6, wherein
the energy output state is a state specified by an integrated value of an output waveform.

8. The medical manipulation apparatus according to any one of claims 1 to 6, wherein
the energy output state is a state specified by a differential value of an output waveform.

9. The medical manipulation apparatus according to any one of claims 1 to 7, wherein
the energy output state is a state specified by an amplitude value of an output waveform.

10. The medical manipulation apparatus according to any one of claims 1 to 7, wherein
the energy output state is a state specified by a frequency of an output waveform.

11. The medical manipulation apparatus according to any one of claims 1 to 10, wherein
the control means detects a state of the energy treatment means, and has a state detecting section which determines an output state.

12. The medical manipulation apparatus according to any one of claims 1 to 10, wherein
the energy treatment means has an output waveform recognition section performing analysis of an energy output waveform.

13. The medical manipulation apparatus according to any one of claims 1 to 10, wherein
the energy treatment means forms a period in which stoppage of output from the energy treatment means or restriction in the predetermined range to the output is performed based on a signal from the control means.
